(19) Europäisches Patentamt
European Patent Office
Office européen des brevets

(11) **EP 0 524 512 B1**

(12) **EUROPÄISCHE PATENTSCHRIFT**

(45) Veröffentlichungstag und Bekanntmachung des Hinweises auf die Patenterteilung:
**03.03.2004** **Patentblatt 2004/10**

(51) Int Cl.[7]: **A61K 31/551**, A61K 31/4184
// (A61K31/551, 31:4184, A61P9:12)

(21) Anmeldenummer: **92111847.7**

(22) Anmeldetag: **11.07.1992**

(54) **Antihypertensive Kombination**

Antihypertensive combinations

Compositions contre l'hypertension

(84) Benannte Vertragsstaaten:
**AT BE CH DE DK ES FR GB GR IT LI LU MC NL PT SE**

(30) Priorität: **23.07.1991** **CH 219191**

(43) Veröffentlichungstag der Anmeldung:
**27.01.1993** **Patentblatt 1993/04**

(73) Patentinhaber: **F. HOFFMANN-LA ROCHE AG**
**4002 Basel (CH)**

(72) Erfinder:
• **Clozel, Jean-Paul**
**F-68300 Saint-Louis (FR)**
• **Müller, Rita**
**CH-4051 Basel (CH)**
• **Osterrieder, Wolfgang**
**W-7889 Grenzach-Wyhlen (DE)**

(74) Vertreter: **Kellenberger, Marcus, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 094 095**    **EP-A- 0 224 810**
**EP-A- 0 268 148**    **EP-A- 0 272 177**

• **ARZNEIMITTELFORSCHUNG Bd. 40, Nr. 4, 1990, Seiten 417 - 421 F. HEFTI 'Antihypertensive properties of the novel calcium antagonist (1S,2S)-2-Ä2 ÄÄ3-(2-benzimidazolyl)propylÜmethylaminoÜethylÜ-6-fluoro-1,2,3,4-tetrahydro-1-isopropyl-2-naphthyl methoxyacetate dihydrochloride in rat models of hypertension.'**
• **F. HEFTI ET AL.: "Antihypertensive properties of the novel calcium antagonist (1S,2S)-2-[2-[[3-(2-benzimidazolyl)propyl]methylamino]ethyl]-6-fluoro-1,2,3,4-tetrahydro-1-iso- propyl-2-naphthyl methoxyacetate dihydrochloride in rat models of hypertension", ARZNEIMITTELFORSCHUNG, , 1990, Band 40, Nr. 4, Seiten 417 - 421**

**Beschreibung**

Die vorliegende Erfindung betrifft pharmazeutische Kombinationspräparate, die zur Behandlung von Hypertension geeignet sind, enthaltend bestimmte Tetrahydronaphthalinderivate und Pyridazodiazepine.

Die angesprochenen Tetrahydronaphthalinderivate sind Calciumantagonisten [EP-A 0.268.148] und sind für die Behandlung von Hypertension geeignet [Clozel et al., Cardiovasc. Drug Rev. 9, 4-17 (1991)].

Die angesprochenen Pyridazodiazepine sind bekannte Hemmer des Angiotensin-konvertierenden Enzyms (ACE) und eignen sich demnach für die Behandlung von Hypertension [EP-A 0.094.095].

Die Behandlung der Hypertension bei gleichzeitiger Applikation des blutdrucksenkenden Calciumantagonisten Nitrendipin und des ACE-Hemmers Captopril [J. Cardiovasc. Pharmacol. 7, S88 - S91 (1985)] hat gezeigt, dass die mit Nitrendipin und Captopril behandelten Patienten auf die gleichzeitige Verabreichung beider Wirkstoffe besser bzw. überhaupt angesprochen haben als auf die alleinige Verabreichung von Captopril bzw. Nitrendipin, wobei in diesen Versuchen beide Einzelkomponenten in der für die jeweilige Monotherapie gebräuchlichen Dosen eingesetzt wurden.

EP-A-0 224 810 beschreibt die synergetisch blutsenkende Wirkung einer Kombination von Ca-Antagonisten des Nitrendipintypus und Pyridazodiazepinen des Cilazapriltypus. EP-A-0 272 177 beschreibt die synergetisch blutdrucksenkende Wirkung einer Kombination des Ca-Antagonisten Diltiazem und von ACE-Hemmem, wie z.B. Cilazapril.

Es besteht aber nach wir vor das Bedürfnis, eine pharmazeutische Kombination bereitzustellen, deren Verabreichung zu einer Senkung des Blutdrucks führt, in welcher die Dosen der Einzelkomponenten signifikant reduziert und unerwünschte Nebenwirkungen, die jeweils bei der notwendigen Dosierung in der Monotherapie auftreten, unterdrückt werden können.

Im Rahmen der vorliegenden Erfindung konnte festgestellt werden, dass bei Verabreichung der erfindungsgemässen Kombination von einem Tetrahydronaphthalinderivat mit einem Pyridazodiazepin die blutdrucksenkenden Eigenschaften der Einzelkomponenten nicht nur addiert, sondern überraschenderweise potenziert werden, wodurch die wirksamen Dosen der beiden Einzelkomponenten signifikant herabgesetzt werden können.

Die erfindungsgemäss antihypertensive Kombination besitzt demnach folgende Vorteile:

a) Die zu applizierenden Wirkstoffmengen werden signifikant reduziert;

b) unerwünschte Nebenwirkungen werden eliminiert bzw. stark reduziert;

c) beide Einzelkomponenten besitzen eine ähnlich lange biologische Halbwertszeit von 10-12 Stunden bei der Behandlung der Hypertension im Menschen. Der Wirkungsverlauf ist daher als gleichmässig zu erwarten;

d) beide Einzelkomponenten weisen eine hohe Bioverfügbarkeit auf (für das Tetrahydronaphthalinderivat [1S,2S]-2-[2-[[3-(2-Benzimidazolyl)propyl]methylamino]äthyl]-6-fluor-1,2,3,4-tetrahydro-1-isopropyl-2-naphthylmethoxyacetat-dihydrochlorid (nachstehend Verbindung A genannt) z.B. 80-100% und für das Pyridazodiazepin 9(S)-[1(S)-Aethoxycarbonyl-3-phenylpropylamino]octahydro-10-oxo-6H-pyridazo[1,2-a][1,2]diazepin-1(S)-carbonsäure (nachstehend Cilazapril genannt) z.B. 70%).

Die Erfindung betrifft demnach neue pharmazeutische Kombinationspräparate zur gleichzeitigen, getrennten oder zeitlich abgestuften Anwendung bei der Behandlung von Hypertension, enthaltend ein Tetrahydronaphthalinderivat, und zwar [1S,2S]-2-[2-[[3-(2-Benzimidazolyl)propyl]-methylamino]äthyl]-6-fluor-1,2,3,4-tetrahydro-1-isopropyl-2-naphthylmethoxyacetat-dihydrochlorid, und ein Pyridazodiazepin, und zwar 9(S)-[1(S)-Aethoxycarbonyl-3-phenylpropylamino]octahydro-10-oxo-6H-pyridazo[1,2-a][1,2]diazepin-1(S)-carbonsäure, wobei die Wirkstoffe entweder in Form ihrer freien Basen, ihrer Hydrate oder ihrer pharmazeutisch verwendbaren Salze vorliegen.

Das Gewichtsverhältnis vom Tetrahydronaphthalinderivat zum Pyridazodiazepin beträgt zweckmässigerweise 100:1 bis 1:1, vorzugsweise 20:1 bis 2:1.

Vorteilhafterweise beträgt die mittels einer Kombination pro Tag zu verabreichende Dosis 5 bis 100 mg eines Tetrahydronaphthalinderivats und 1 bis 5 mg eines Pyridazodiazepins. Im allgemeinen beträgt die täglich zu verabreichende Gesamtmenge von einem Tetrahydronaphthalinderivat und einem Pyridazodiazepin maximal 55 mg. Wird ein Hydrat oder ein pharmazeutisch verwendbares Salz eingesetzt, so sind die obigen Werte entsprechend zu ändern.

Gegenstand der vorliegenden Erfindung sind somit

- eine Kombination vom Tetrahydronaphthalinderivat und dem Pyridazodiazepin

- eine pharmazeutische Zubereitung, enthaltend das Tetrahydronaphthalinderivat und das Pyridazodiazepin

- die Herstellung einer pharmazeutischen Zubereitung, welche dadurch gekennzeichnet ist, dass man ein Gemisch vom Tetrahydronaphthalinderivat und dem Pyridazodiazepin in eine galenische Darreichungsform bringt

- die Verwendung einer Kombination vom Tetrahydronaphthalinderivat und dem Pyridazodiazepin bzw. einer pharmazeutischen Zubereitung, enthaltend das Tetrahydronaphthalinderivat und das Pyridazodiazepin zur Bekämpfung bzw. Verhütung von Krankheiten, insbesondere von Kreislauferkrankungen, ganz besonders bei der Bekämpfung bzw. Verhütung von Hypertension und deren Folgeerkrankungen.

Bei dem Tetrahydronaphthalinderivate handelt es sich um:
[1S,2S]-2-[2-[[3-(2-Benzimidazolyl)propyl]methylamino]äthyl]-6-fluor-1,2,3,4-tetrahydro-1-isopropyl-2-naphthylmethoxyacetat-dihydrochlorid (Verbindung A).

Der geeignetste Vertreter aus der Gruppe der Pyridazodiazepine ist das Cilazapril als soches oder als Hydrobromid oder Hydrat.

Mit der erfindungsgemässen Kombination kann mit geringen Wirkstoffdosen eine regelmässige und lang andauernde blutdrucksenkende Wirkung erzielt werden.

Die vorteilhafte überadditive blutdrucksenkende Wirkung der erfindungsgemässen Kombination gegenüber derjenigen der beiden Einzelkomponenten soll anhand des nachfolgend wiedergegebenen Versuchs gezeigt werden.

Die antihypertensive Wirkung der Kombination wurde an wachen Hunden mit renalem Bluthochdruck untersucht. Der Hochdruck wurde an Deutschen Schäferhunden (Gewicht 23-30 kg) mit der bekannten Methode erzeugt, bei der eine Niere mit Zellophan umwickelt und in der Arteria renalis der kontralateralen Niere mit Hilfe eines Okkluders eine Stenose gebildet wird. Der Blutdruck wurde mit einem in die abdominale Aorta implantiertem Katheter gemessen, der an einen Sender in der Bauchhöhle angeschlossen war (Telemetrie).

Die Abbildung 1 illustriert die Wirkung von Cilazapril (10 mg/kg per os) und Verbindung A (30 mg/kg per os) allein, sowie die Wirkung der gleichzeitigen Verabreichung derselben Dosis der beiden Substanzen (n = 3 Hunde für Cilazapril und die Kombination, n = 4 Hunde für Verbindung A). Cilazapril allein war ohne Effekt, und Verbindung A senkte den Blutdruck (MAP) um 10-20 mmHg. Die Kombination war zu allen Zeitpunkten wesentlich wirksamer.

Die synergistische Wirkung der Kombination war auch bei einer weiteren Versuchsreihe offensichtlich, in der die Dosierung der Komponenten niedriger war, nämlich 3 mg/kg per os für Cilazapril und 10 mg/kg per os für Verbindung A (selbe Zahl von Versuchstieren). Das Resultat dieser Versuchsreihe ist in der Abbildung 2 dargestellt.

Abb. 1

ΔMAP (mmHg)

Zeit (Stunden)

−ㅁ− Verbindung A   −△− Cilazapril   −o− Kombination

EP 0 524 512 B1

Abb. 2

Die vorteilhafte überadditive Wirkung der erfindungsgemässen Kombination gegenüber derjenigen der beiden Einzelkomponenten bei der bekannten Rückbildung der durch chronischen Bluthochdruck bedingten Hypertrophie der Media in den grossen, muskulären Arterien durch Behandlung des Bluthochdrucks mit ACE-Hemmern sowie anderen gängigen Therapeutika [Hypertension 9, 178-187 (1987)] kann mit Hilfe des nachstehend beschriebenen Versuchs gezeigt werden.

Der Einfluss der Einzelverbindungen und ihrer Kombination auf die Blutgefässe wurde an Ratten untersucht. Männliche Ratten, Stamm: RoRo (Gewicht ca. 400 g; 4-5 Monate alt; Institut für Biologisch-Medizinische Forschung, Füllinsdorf, CH) wurden verwendet. Die Tiere wurden randomisiert in eine Kontrollgruppe und in Behandlungsgruppen eingeteilt. Die Dauer der Behandlung war 15 Tage. Cilazapril wurde in einer solchen Menge dem Futter beigemischt, dass die tägliche Aufnahme durchschnittlich 10 mg/kg betrug, und Verbindung A (30 mg/kg) wurde mit einer Schlundsonde verabreicht. Die Kontrolltiere erhielten das gleiche Laborfutter ohne Zusatz.

Nach 15 Tagen wurden die Ratten mit Aether anaesthesiert und die Arteria carotis durch Perfusion mit Fixationsmittel (2,5% Glutaraldehyd in 0,1 M Phosphatpuffer, pH 7,4) fixiert. Hierzu wurde eine Sonde durch den linken Herzventrikel in die aufsteigende Aorta geführt (Einfluss), und eine zweite Sonde durch den rechten Ventrikel in den Vorhof geschoben (Abfluss). Das Gefäss-System wurde zunächst mit 10 ml gepuffterter isotonischer NaCl-Lösung gespült und dann 15 Minuten lang mit Fixationsmittel bei einem Druck von 11,7 kPa fixiert. Anschliessend wurde die rechte Arteria carotis herauspräpariert, von anhaftendem Gewebe befreit und zur weiteren Fixation in 2,5% Glutaraldehyd eingelegt. Jede Arterie wurde vom distalen zum proximalen Ende in fünf Gefässegmente aufgeteilt, entwässert und in EPON 12 (eingetragene Marke der Shell A.G.) eingebettet. Das mittlere Segment wurde für die morphologischen Untersuchungen verwendet. Semidünne Querschnitte (1 μm dick) wurden mit Toluidinblau und basischem Fuchsin gefärbt. Die Querschnittsfläche der Media wurde mit dem Morphometrie-System DIASYS (Datalab, Heinz Meyer, CH-3367 Thörigen) gemessen.

Die Fläche der Media bei den Kontrolltieren war $89000 \pm 5000 \ \mu m^2$. Die Einzelbehandlung war ohne Wirkung ($84000 \pm 11000 \ \mu m^2$ bei Cilazapril und $87000 \pm 4000 \ \mu m^2$ bei Verbindung A). (Siehe auch Abbildung 3, die Zahlen in den Balken geben die Zahl der Versuchstiere in den jeweiligen Gruppen an). Die Kombination senkte die Mediafläche um 15% auf $74000 \pm 4000 \ \mu m^2$ (statistisch signifikant; p <0,05 mit dem t-Test nach Student).

Die Abnahme der Mediafläche durch die Kombination wurde in einer zweiten Versuchsreihe bestätigt.

Offensichtlich besteht zwischen ACE-Hemmern und Calciumantagonisten eine mechanistische Interaktion. Es ist bekannt, dass durch die Verabreichung eines Calciumantagonisten und der daraus resultierenden Blutdrucksenkung eine kompensatorische Stimulierung des Renin-Angiotensin-Systems erfolgt. Diese Kompensation wird durch die Anwendung des ACE-Hemmers unterdrückt.

Abb. 3

Die vorstehenden Ergebnisse zeigen die unerwartet vorteilhaften Eigenschaften der erfindungsgemässen Kombinationen. Bei Kenntnis des Standes der Technik konnte nicht erwartet werden, dass gerade die Kombination von Tetrahydronaphthalinderivaten, insbesondere von Verbindung A, mit Pyridazodiazepinen, insbesondere mit Cilazapril, eine so optimale blutdrucksenkende Wirkung zeigt.

Die erfindungsgemässen Kombinationen werden im allgemeinen oral, z.B. in Form von Tabletten, Lacktabletten, Dragées, Hart- und Weichgelatinekapseln, Lösungen, Emulsionen oder Suspensionen verabreicht. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln kann eine erfindungsgemässe Kombination mit pharmazeutisch inerten, anorganischen oder organischen Excipientien verarbeitet werden. Als solche Excipientien kann man z.B. für Tabletten, Dragées und Hartgelatinekapseln Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.; je nach Beschaffenheit des Wirkstoffes sind jedoch bei Weichgelatinekapseln überhaupt keine Excipientien erforderlich.

Zur Herstellung von Lösungen und Sirupen eignen sich als Excipientien z.B. Wasser, Polyole, Saccharose, Inverzucker, Glukose und dergleichen.

Für Injektionslösungen eignen sich als Excipientien z.B. Wasser, Alkohole, Polyole, Glyzerin, vegetabile Oele etc.

Für Suppositorien eignen sich als Excipientien z.B. natürliche oder gehärtete Oele, Wachse, Fette, halbflüssige oder flüssige Polyole und dergleichen.

Die pharmazeutischen Zubereitungen können daneben noch Konservierungsnaittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutische wertvolle Stoffe enthalten.

Die nachfolgenden Beispiele erläutern die Erfindung.

Beispiel 1

Herstellung von Lacktabletten folgender Zusammensetzung:

| Lacktablettenkern: | |
| --- | --- |
| a. Verbindung A | 29.07 mg (= 25 mg Base) |
| b. Cilazapril | 1.25 mg |
| c. Lactose wasserfrei | 70.18 mg |
| d. Maisstärke weiss | 30.00 mg |
| e. Polyvinylpyrrolidon | 5.00 mg |
| f. Talk | 5.00 mg |
| g. Natriumstearylfumarat | 1.50 mg |
| Gewicht pro Lacktablettenkern | 142.00 mg |

| Lacküberzug: | |
| --- | --- |
| h. Hydroxypropylmethylcellulose | 4.00 mg |
| i. Polyäthylenglykol 6000 | 1.00 mg |
| j. Titandioxid | 1.60 mg |
| k. Talk | 1.40 mg |
| Gewicht Lacküberzug | 8.00 mg |
| Totalgewicht pro Lacktablette | 150.00 mg |

Herstellungsverfahren:

Herstellung des Lacktablettenkerns:

b wird sukzessive und portionsweise mit c homogen vermischt und gesiebt. Dann werden a, d und e zugegeben und das Ganze kurz gemischt, gesiebt und während geeigneter Zeit im Planetenmischer befeuchtet. Die befeuchtete Masse wird durch ein geeignetes Sieb granuliert, getrocknet und anschliessend durch ein geeignetes Sieb gebrochen. Dazu werden die gesiebten f und g zugegeben und homogen gemischt. Die pressfertige Mischung wird zu Lacktablettenkernen geeigneter Grösse und Form (mit oder ohne Bruchrille) à 142.0 mg verpresst.

Herstellung des Lacküberzugs:

Aus h bis k wird eine wässrige Lacksuspension hergestellt und damit die Lacktablettenkerne in geeignete Weise mit Hilfe eines Lackierverfahrens in einem Dragierkessel oder einer anderen Lackierapparatur überzogen, bis die Lacktabletten ein Endgewicht von 150 mg erreicht haben.

Beispiel 2

Herstellung von Hartgelatinekapseln folgender Zusammensetzung:

| a. Verbindung A | 29.07 mg (= 25 mg Base) |
| --- | --- |
| b. Cilazapril | 2.50 mg |
| c. Lactose pulv. | 26.93 mg |

(fortgesetzt)

| | |
|---|---|
| d. Lactose krist. | 60.00 mg |
| e. Microkristalline Cellulose | 50.00 mg |
| f. Talk | 10.00 mg |
| g. Natriumstearylfumarat | 1.50 mg |
| Füllgewicht pro Kapsel | 180.00 mg |

Herstellungsverfahren:

b wird sukzessive und portionsweise mit c homogen vermischt, gesiebt, und die gesiebten a, d und e zugefügt und in geeigneter Weise gemischt. Dazu werden die gesiebten f und g zugegeben und während geeigneter Zeit gemischt. Die abfüllfertige Endmischung wird in Hartgelatinekapseln geeigneter Grösse und Farbe abgefüllt.

Beispiel 3

Herstellung von Lacktabletten CR (controlled release) folgender Zusammensetzung:

| CR-Lacktablettenkern: | |
|---|---|
| a. Verbindung A | 58.13 mg (= 50 mg Base) |
| b. Cilazapril | 2.50 mg |
| c. Lactose wasserfrei | 45.37 mg |
| d. Methocel (eingetragene Marke der Dow Chemical Company) | 10.00 mg |
| e. Hydroxypropylcellulose | 10.00 mg |
| f. Talk | 4.00 mg |
| g. Natriumstearylfumarat | 2.00 mg |
| Gewicht pro CR-Lacktablettenkern | 132.00 mg |

| Lacküberzug: | |
|---|---|
| h. Hydroxypropylmethylcellulose | 4.00 mg |
| i. Polyäthylenglykol 6000 | 1.00 mg |
| j. Titandioxid | 1.60 mg |
| k. Talk | 1.40 mg |
| Gewicht Lacküberzug | 8.00 mg |
| Totalgewicht pro Lacktablette CR | 140.00 mg |

Herstellungsverfahren:

Herstellung des CR-Lacktablettenkerns:

b wird sukzessive und portionsweise mit c homogen gemischt und gesiebt, und anschliessend werden die gesiebten a, d und e homogen zugemischt und auf einem Walzen-Kompaktor in geeigneter Weise kompaktiert. Das kompaktierte Material wird durch ein geeignetes Sieb gesiebt und anschliessend mit einer gesiebten Mischung aus f und g homogen gemischt und zu Tablettenkernen à 132,0 mg in geeigneter Grösse und Form verpresst.

Herstellung des Lacküberzugs:

Aus h bis k wird eine wässrige Lacksuspension hergestellt und damit die Lacktablettenkerne in geeigneter Weise mit Hilfe eines Lackierverfahrens in einem Dragierkessel oder einer anderen Lackierapparatur überzogen, bis die Lacktabletten ein Endgewicht von 140 mg erreicht haben.

Beispiel 4

Herstellung von CR-Pelletformulierung in Hartgelatinekapsel folgender Zusammensetzung:

| Pellets: | |
|---|---|
| a. Verbindung A | 58.13 mg (= 50 mg Base) |
| b. Cilazapril | 2.50 mg |
| c. Microkristalline Cellulose | 139.37 mg |
| Gewicht Pellet-Kerne pro Kapsel | 200.00 mg |

| Pellet-CR-Lack: | |
|---|---|
| d. Aethylcellulose (AQUACOAT-Dispers.) | 16.00 mg |
| e. Dibutylsebacat | 4.00 mg |
| Gew. Lack./Pellet-Kerne/Kapsel | 20.00 mg |
| Totalgewicht CR-Pellets pro Kapsel | 220.00 mg |

Herstellungsverfahren:

Herstellung der Pellet-Kerne:

a, b und c werden in geeigneter Weise homogen miteinander gemischt, mit der geeigneten Menge Wasser in einem Mischer befeuchtet und durch eine geeignete Lochscheibe extrudiert. Die extrudierte Masse wird in einem Spheronizer gebrochen, ausgerundet und anschliessend im Wirbelbett getrocknet.

Herstellung des Lacküberzugs:

Die Pellet-Kerne werden in einem kontinuierlichen Prozess mit einem Wirbelschicht-Sprühverfahren unter geeigneten Bedingungen mit der wässrigen Dispersion von d und e überzogen bis der Lacküberzug 10% des Pellets-Kerngewichts beträgt. Anschliessend erfolgt eine Wärmenachbehandlung der überzogenen CR-Pellets und die Abfüllung ä 220 mg in Hartgelatinekapseln geeigneter Grösse und Farbe.

**Patentansprüche**

**Patentansprüche für folgende Vertragsstaaten : AT, BE, CH, DE, DK, FR, GB, IT, LI, LU, MC, NL, PT, SE**

1. Pharmazeutisches Kombinationspräparat, **dadurch gekennzeichnet, dass** es ein Tetrahydronaphthalinderivat, und zwar [1S,2S]-2-[2-[[3-(2-Benzimidazolyl)propyl]-methylamino]äthyl]-6-fluor-1,2,3,4-tetrahydro-1-isopropyl-2-naphthylmethoxyacetat-dihydrochlorid, und ein Pyridazodiazepin, und zwar 9(S)-[1(S)-Aethoxycarbonyl-3-phenylpropylamino]octahydro-10-oxo-6H-pyridazo[1,2-a][1,2]diazepin-1(S)-carbonsäure, in Form ihrer freien Basen, ihrer Hydrate oder ihrer pharmazeutisch verwendbaren Salze enthält.

2. Präparat nach Anspruch 1, **dadurch gekennzeichnet, dass** das Gewichtserhältnis vom Tetrahydronaphthalinderivat zum Pyridazodiazepin 100:1 bis 1:1 in Bezug auf freie Base(n) beträgt.

3. Präparat nach Anspruch 2, **dadurch gekennzeichnet, dass** das Gewichtserhältnis 20:1 bis 2:1 beträgt.

4. Präparat nach einem der Ansprüche 1 bis 3, **dadurch gekennzeichnet, dass** es 5 bis 100 mg des Tetrahydronaphthalinderivates und 1 bis 5 mg des Pyridazodiazepins oder äquivalente Mengen eines Hydrates oder eines pharmazeutisch verwendbaren Salzes enthält.

5. Präparat nach einem der Ansprüche 1 bis 4, **dadurch gekennzeichnet, dass** das Gesamtgewicht vom Tetrahydronaphthalinderivat und vom Pyridazodiazepin maximal 55 mg in Bezug auf freie Base(n) beträgt.

**6.** Präparat nach einem der Ansprüche 1 bis 5, **dadurch gekennzeichnet, dass** die 9(S)-[1(S)-Aethoxycarbonyl-3-phenylpropylamino]octahydro-10-oxo-6H-pyridazo-[1,2-a][1,2]diazepin-1(S)-carbonsäure als Salz oder Hydrat vorliegt.

**7.** Präparat nach Anspruch 6, **dadurch gekennzeichnet, dass** die 9(S)-[1(S)-Aethoxycarbonyl-3-phenylpropylamino]octahydro-10-oxo-6H-pyridazo[1,2-a][1,2]-diazepin-1(S)-carbonsäure als Hydrobromid oder Hydrat vorliegt.

**8.** Präparat gemäss einem der Ansprüche 1 bis 7, zur Anwendung bei der Bekämpfung bzw. Verhütung von Hypertension und deren Folgeerkrankungen.

**9.** Präparat gemäss einem der Ansprüche 1 bis 8, zur gleichzeitigen, getrennten oder zeitlich abgestuften Anwendung in der Bekämpfung bzw. Verhütung von Kreislauferkrankungen, insbesondere von Hypertension und deren Folgeerkrankungen.

**10.** Verfahren zur Herstellung eines Präparates gemäss einem der Ansprüche 1 bis 9, **dadurch gekennzeichnet, dass** man ein Gemisch der beiden Wirkstoffe in eine galenische Darreichungsform bringt.

**11.** Verwendung von [1S,2S]-2-[2-[[3-(2-Benzimidazolyl)propyl]methylamino]äthyl]-6-fluor-1,2,3,4-tetrahydro-1-isopropyl-2-naphthylmethoxyacetatdihydrochlorid in Kombination mit 9(S)-[1(S)-Aethoxycarbonyl-3-phenylpropylamino]octahydro-10-oxo-6H-pyridazo[1,2-a][1,2]diazepin-1(S)-carbonsäure zur Herstellung eines Arzneimittels für eine Antihypertensive Anwendung.

**Patentansprüche für folgende Vertragsstaaten : ES, GR**

**1.** Verfahren zur Herstellung eines pharmazeutischen Kombinationspräparates, **dadurch gekennzeichnet, dass** man ein Gemisch eines Tetrahydronaphthalinderivates, und zwar des [1S,2S]-2-[2-[[3-(2-Benzimidazolyl)propyl]methylamino]äthyl]-6-fluor-1,2,3,4-tetrahydro-1-isopropyl-2-naphthylmethoxyacetats-dihydrochlorid, und eines Pyridazodiazepins, und zwar der 9(S)-[1(S)-Aethoxycarbonyl-3-phenylpropylamino]octahydro-10-oxo-6Hpyridazo[1,2-a][1,2]diazepin-1(S)-carbonsäure, in Form ihrer freien Basen, ihrer Hydrate oder ihrer pharmazeutisch verwendbaren Salze in eine galenische Darreichungsform bringt.

**2.** Verfahren nach Anspruch 1, **dadurch gekennzeichnet, dass** das Gewichtsverhältnis vom Tetrahydronaphthalinderivat zum Pyridazodiazepin 100:1 bis 1:1 in Bezug auf freie Base(n) beträgt.

**3.** Verfahren nach Anspruch 2, **dadurch gekennzeichnet, dass** das Gewichtsverhältnis 20:1 bis 2:1 beträgt.

**4.** Verfahren nach einem der Ansprüche 1 bis 3, **dadurch gekennzeichnet, dass** ein Gemisch von 5 bis 100 mg des Tetrahydronaphthalinderivates und 1 bis 5 mg des Pyridazodiazepins oder äquivalente Mengen eines Hydrates oder eines pharmazeutisch verwendbaren Salzes zu einer Tagesdosis verarbeitet wird.

**5.** Verfahren nach einem der Ansprüche 1 bis 4, **dadurch gekennzeichnet, dass** ein Gemisch vom Tetrahydronaphthalinderivat und vom Pyridazodiazepin von maximal 55 mg in Bezug auf freie Base(n) zu einer Tagesdosis verarbeitet wird.

**6.** Verfahren nach einem der Ansprüche 1 bis 5, **dadurch gekennzeichnet, dass** die 9(S)-[1(S)-Aethoxycarbonyl-3-phenylpropylamino]octahydro-10-oxo-6H-pyridazo[1,2-a][1,2]diazepin-1(S)-carbonsäure als Salz oder Hydrat vorliegt.

**7.** Verfahren nach Anspruch 6, **dadurch gekennzeichnet, dass** die 9(S)-[1(S)-Aethoxycarbonyl-3-phenylpropylamino]octahydro-10-oxo-6H-pyridazo[1,2-a][1,2]diazepin-1(S)-carbonsäure als Hydrobromid oder Hydrat vorliegt.

**8.** Verwendung von [1S,2S]-2-[2-[[3-(2-Benzimidazolyl)propyl]methylamino]äthyl]-6-fluor-1,2,3,4-tetrahydro-1-isopropyl-2-naphthylmethoxyacetatdihydrochlorid in Kombination mit 9(S)-[1(S)-Aethoxycarbonyl-3-phenylpropylamino]octahydro-10-oxo-6H-pyridazo[1,2-a][1,2]diazepin-1(S)-carbonsäure zur Herstellung eines Arzneimittels für eine Antihypertensive Anwendung.

**Claims**

**Claims for the following Contracting States : AT, BE, CH, DE, DK, FR, GB, IT, LI, LU, MC, NL, PT, SE**

1.  A pharmaceutical combination preparation, **characterized in that** it contains a tetrahydronaphthalene derivative, namely [1S,2S]-2-[2-[[3-(2-benzimidazolyl)propyl]methylamino]ethyl]-6-fluoro-1,2,3,4-tetrahydro-1-isopropyl-2-naphthylmethoxyacetate dihydrochloride, and a pyridazodiazepine, namely 9(S)-[1(S)-ethoxycarbonyl-3-phenylpropylamino]octahydro-10-oxo-6H-pyridazo[1,2-a][1,2]diazepine-1(S)-carboxylic acid, in the form of their free bases, their hydrates or their pharmaceutically usable salts.

2.  A preparation according to claim 1, **characterized in that** the weight ratio of tetrahydronaphthalene derivative to pyridazodiazepine is 100:1 to 1:1 with respect to free base(s).

3.  A preparation according to claim 2, **characterized in that** the weight ratio is 20:1 to 2:1.

4.  A preparation according to any one of claims 1 to 3, **characterized in that** it contains 5 to 100 mg of the tetrahydronaphthalene derivative and 1 to 5 mg of the pyridazodiazepine or equivalent amounts of a hydrate or of a pharmaceutically usable salt.

5.  A preparation according to any one of claims 1 to 4, **characterized in that** the total weight of tetrahydronaphthalene derivative and of pyridazodiazepine is a maximum 55 mg with respect to free base(s).

6.  A preparation according to any one of claims 1 to 5, **characterized in that** the 9(S)-[1(S)-ethoxycarbonyl-3-phenylpropylamino]octahydro-10-oxo-6H-pyridazo[1,2-a]-[1,2]diazepine-1(S)-carboxylic acid is present as a salt or hydrate.

7.  A preparation according to claim 6, **characterized in that** the 9(S)-[1(S)-ethoxycarbonyl-3-phenylpropylamino] octahydro-10-oxo-6H-pyridazo[1,2-a][1,2]diazepine-1(S)-carboxylic acid is present as the hydrobromide or hydrate.

8.  A preparation in accordance with any one of claims 1 to 7 for use in the control or prevention of hypertension and disorders stemming therefrom.

9.  A preparation in accordance with any one of claims 1 to 8 for the simultaneous, separate or planned stepwise use in the control or prevention of circulatory disorders, especially of hypertension and disorders stemming therefrom.

10. A process for the manufacture of a preparation in accordance with any one of claims 1 to 9, **characterized by** bringing a mixture of the two active substances into a galenical administration form.

11. The use of [1S,2S]-2-[2-[[3-(2-benzimidazolyl)propyl]methylamino]ethyl]-6-fluoro-1,2,3,4-tetrahydro-1-isopropyl-2-naphthylmethoxyacetate dihydrochloride in combination with 9(S)-[1(S)-ethoxycarbonyl-3-phenylpropylamino] octahydro-10-oxo-6H-pyridazo[1,2-a][1,2]diazepine-1(S)-carboxylic acid for the manufacture of a medicament for an antihypertensive use.

**Claims for the following Contracting States : ES, GR**

1.  A process for the manufacture of a pharmaceutical combination preparation, **characterized by** bringing a mixture of a tetrahydronaphthalene derivative, namely [1S,2S]-2-[2-[[3-(2-benzimidazolyl)propyl]methylamino]ethyl]-6-fluoro-1,2,3,4-tetrahydro-1-isopropyl-2-naphthylmethoxyacetate dihydrochloride, and a pyridazodiazepine, namely 9(S)-[1(S)-ethoxycarbonyl-3-phenylpropylamino]octahydro-10-oxo-6H-pyridazo[1,2-a][1,2]-diazepine-1 (S)-carboxylic acid, in the form of their free bases, their hydrates or their pharmaceutically usable salts into a galenical administration form.

2.  A process according to claim 1, **characterized in that** the weight ratio of tetrahydronaphthalene derivative to pyridazodiazepine is 100:1 to 1:1 with respect to free base(s).

**3.** A process according to claim 2, **characterized in that** the weight ratio is 20:1 to 2:1.

**4.** A process according to any one of claims 1 to 3, **characterized in that** a mixture of 5 to 100 mg of the tetrahydronaphthalene derivative and 1 to 5 mg of the pyridazodiazepine or equivalent amounts of a hydrate or of a pharmaceutically usable salt is processed for a daily dosage.

**5.** A process according to any one of claims 1 to 4, **characterized in that** a mixture of tetrahydronaphthalene derivative and of pyridazodiazepine of a maximum 55 mg with respect to free base(s) is processed for a daily dosage.

**6.** A process according to any one of claims 1 to 5, **characterized in that** the 9(S)-[1(S)-ethoxycarbonyl-3-phenylpropylamino]octahydro-10-oxo-6H-pyridazo[1,2-a][1,2]-diazepine-1(S)-carboxylic acid is present as a salt or hydrate.

**7.** A process according to claim 6, **characterized in that** the 9(S)-[1(S)-ethoxycarbonyl-3-phenylpropylamino]octahydro-10-oxo-6H-pyridazo[1,2-a][1,2]diazepine-1(S)-carboxylic acid is present as the hydrobromide or hydrate.

**8.** The use of [1S,2S]-2-[2-[[3-(2-benzimidazolyl)propyl]methylamino]ethyl]-6-fluoro-1,2,3,4-tetrahydro-1-isopropyl-2-naphthylmethoxyacetate dihydrochloride in combination with 9(S)-[1(S)-ethoxycarbonyl-3-phenylpropylamino]octahydro-10-oxo-6H-pyridazo[1,2-a][1,2]diazepine-1(S)-carboxylic acid for the manufacture of a medicament for an antihypertensive use.

**Revendications**

**Revendications pour les Etats contractants suivants : AT, BE, CH, DE, DK, FR, GB, IT, LI, LU, MC, NL, PT, SE**

**1.** Préparation pharmaceutique combinée, **caractérisée en ce qu'**elle contient un dérivé de la tétrahydronaphtalène, à savoir le [1S,2S]-2-[2-[[3-(2-benzimidazolyl)propyl]-méthylamino]éthyl]-6-fluor-1,2,3,4-tétrahydro-1-isopropyl-2-naphthylméthoxyacétate-dihydrochlorure, et une pyridazodiazépine, à savoir le 9(S)-1[1(S)-éthoxycarbonyl-3-phénylpropylamino]octahydro-10-oxo-6H-pyridazo[1,2-a][1,2]-diazépine-1(S)-acide carbonique, sous la forme de leurs bases libres, leurs hydrates ou leurs sels utilisables en pharmacie.

**2.** Préparation selon la revendication 1, **caractérisée en ce que** le rapport du poids du dérivé de tétrahydronaphtalène et du poids de la pyridazodiazépine est de 100:1 à 1:1 en ce qui concerne la/les) base(s) libre(s).

**3.** Préparation selon la revendication 2, **caractérisée en ce que** le rapport des poids est de 20:1 à 2:1.

**4.** Préparation selon l'une des revendications 1 à 3, **caractérisée en ce qu'**elle contient 5 à 100 mg du dérivé de tétrahydronaphtalène et de 1 à 5 mg de pyridazodiazépine ou des quantités équivalentes d'un hydrate ou d'un sel utilisable en pharmacie.

**5.** Préparation selon l'une des revendications 1 à 4, **caractérisée en ce que** le poids total du dérivé de tétrahydronaphtalène et de la pyridazodiazépine est de 55 mg au maximum en ce qui concerne la/les) base(s) libre(s).

**6.** Préparation selon l'une des revendications 1 à 5, **caractérisée en ce que** le 9(S)-[1(S)-éthoxycarbonyl-3-phénylpropylamino]octahydro-10-oxo-6H-pyridazo[1,2-a] [1,2]diazépine-1(S)-acide carbonique existe sous la forme de sel ou d'hydrate.

**7.** Préparation selon la revendication 6, **caractérisée en ce que** le 9(S)-1[1(S)-éthoxycarbonyl-3-phénylpropylamino]octahydro-10-oxo-6H-pyridazo[1,2-a] [1,2]-diazépine-1(S)-acide carbonique existe sous la forme d'hydrobromure ou d'hydrate.

**8.** Préparation selon l'une des revendications 1 à 7, destinée à être utilisé pour combattre ou empêcher l'hypertension et les pathologies qui en découlent.

**9.** Préparation selon l'une des revendications 1 à 8, pour une utilisation simultanée, séparée ou échelonnée dans le temps pour combattre et/ou empêcher les maladies cardio-vasculaires, en particulier l'hypertension et les pathologies qui en découlent.

10. Procédé de fabrication d'une préparation selon l'une des revendications 1 à 9, **caractérisée en ce que** l'on transforme un mélange des deux substances actives en une forme d'administration galénique.

11. Utilisation du [1S,2S]-2-[2-[[3-(2-benzimidazolyl)propyl]-méthylamino]éthyl]-6-fluor-1,2,3,4-tétrahydro-1-isopropyl-2-naphthylméthoxyacétate-dihydrochlorure en association avec le 9(S)-[1(S)-éthoxycarbonyl-3-phénylpropylamino]octahydro-10-oxo-6H-pyridazo[1,2-a][1,2]diazépine-1(S)-acide carbonique pour fabriquer un médicament destiné à être utilisé comme antihypertenseur.

**Revendications pour les Etats contractants suivants : ES, GR**

1. Procédé de fabrication d'une préparation pharmaceutique combinée, **caractérisée en ce que** l'on transforme en une forme d'administration galénique un mélange d'un dérivé de la tétrahydronaphtalène, à savoir [1S,S]-2 [2-[[3-(2-benzimidazolyl)propyl]-méthylamino]éthyle]-6-fluor-1,2,3,4-tétrahydro-1-isopropyl-2-naphthylméthoxyacétate-dihydrochlorure, et d'une pyridazodiazépine, à savoir 9(S)-[1(S)-éthoxycarbonyl-3-phénylpropylaminoloctahydro-10-oxo-6H-pyridazo[1,2-a][1,2]diazépine-1(S)-acide carbonique, sous la forme de leurs bases libres, de leurs hydrates ou de leurs sels utilisables en pharmacie.

2. Procédé selon la revendication 1, **caractérisé en ce que** le rapport du poids du dérivé de tétrahydronaphtalène et du poids de la pyridazodiazépinc est de 100:1 à 1:1 en ce qui concerne la/les) base(s) libre(s).

3. Procédé selon la revendication 2, **caractérisé en ce que** le rapport des poids est de 20:1 à 2:1.

4. Procédé selon l'une des revendications 1 à 3, **caractérisé en ce qu'**un mélange de 5 à 100 mg du dérivé de tétrahydronaphtalène et 1 à 5 mg de pyridazodiazépine ou quantités équivalentes d'un hydrate ou d'un sel utilisable en pharmacie est transformé en dose quotidienne.

5. Procédé selon l'une des revendications 1 à 4, **caractérisé en ce qu'**un mélange de dérivé de tétrahydronaphtalène et de pyridazodiazépine de 55 mg au maximum en ce qui concerne la (les) base(s) libre(s) est transformé en une dose quotidienne.

6. Procédé selon l'une des revendications 1 à 5, **caractérisé en ce que** le 9(S)-[1(S)-éthoxycarbonyl-3-phénylpropylamino]octahydro-10-oxo-6H-pyridazo[1,2-a][1,2]diazépine-1(S)-acide carbonique existe sous forme de sel ou d'hydrate.

7. Procédé selon la revendication 6, **caractérisé en ce que** le 9(S)-[1(S)-éthoxycarbonyl-3-phénylpropylamino]octahydro-10-oxo-6H-pyridazo[1,2-a][1,2]diazépine-1(S)-acide carbonique existe sous forme d'hydrobromure ou d'hydrate.

8. Utilisation du [1S,S]-2-[2-[[3-(2-benzimidazolyl)propyl]-méthylamino]éthyl]-6-fluor-1,2,3,4-tétrahydro-1-isopropyl-2-naphthylméthoxyacétate-dihydrochlorure associé au 9(S)-[1(S)-éthoxycarbonyl-3-phénylpropylamino]octahydro-10-oxo-6H-pyridazo[1,2-a][1,2]diazépine-1(S)-acide carbonique pour fabriquer un médicament utilisé comme antihypertenseur.